# EUROPEAN PATENT APPLICATION

(11) **EP 4 148 707 A1**
(43) Date of publication of application: **15.03.2023**
(21) Application number: 21800079.2
(22) Date of filing: 07.05.2021
(51) Int. Cl.: G09B 19/00, A61B 3/11, A61B 3/113, G06F 3/01, A61B 5/11

(54) **THREE-DIMENSIONAL COGNITIVE ABILITY EVALUATION SYSTEM**

(30) Priority: 08.05.2020 JP 2020082634
(71) Applicant: Sumitomo Pharma Co., Ltd., Osaka 541-0045 (JP)
(72) Inventor: OCHIAI Yasushi, Tokyo 103-6012 (JP); KASAI Kazuki, Tokyo 158-0094 (JP)
(74) Representative: Reeve, Nicholas Edward
(86) International application number: PCT/JP2021/017539
(87) International publication number: WO 2021/225166

(57) **Abstract**

There are provided a system, an apparatus, a program, and a method for enabling objective evaluation of a three-dimensional cognitive ability through quantification. A three-dimensional cognitive ability of a measurement target person is evaluated by acquiring a position of a moving object, the position enabling identification of a distance between the moving object and the measurement target person, by receiving input of an active response of the measurement target person taken in response to a three-dimensional position of the object recognized by the measurement target person, and by determining whether the position of the object that is acquired and the response that is input correctly match. The moving object may be provided in virtual reality by a virtual reality headset, and the three-dimensional cognitive ability may also be evaluated by displaying a moving image of when the object seen from a predetermined point of view is moved in virtual reality from a movement start position to a movement end position, in a direction of approaching the predetermined point of view along a predetermined movement route, and based on a response corresponding to a position of the object.

## Description

### Technical Field

The present invention relates to a three-dimensional cognitive ability evaluation system for evaluating a three-dimensional cognitive ability based on a response to an object.

### Background Art

A three-dimensional cognitive ability is an ability of recognizing distance/depth of an object and of appropriately coping with the same. In recent years, there are observations that, when a cognitive function declines, the three-dimensional cognitive ability of an aged person or the like declines as well, and thus, it is likely that evaluating the three-dimensional cognitive ability achieves a similar effectiveness as evaluating the cognitive function. However, it is difficult to objectively evaluate the cognitive function. Accordingly, objective evaluation of the cognitive function through quantification of the three-dimensional cognitive function, for example, contributes to objective evaluation of the cognitive function, and is very advantageous. However, there is no technique available for quantifying the three-dimensional cognitive ability.

The three-dimensional cognitive ability is premised on normal visual functions (such as pupil modulation and eye movement) for acquiring visual information, and is defined as an ability to accurately grasp, by a brain, a positional relationship to an object at the time of seeing a moving object or the like, based on visual information about the moving object, and to take an appropriate, accurate action on the moving object based on the grasped positional relationship.

As methods for checking the normal visual function, methods such as pupillometry and near point distance measurement are known, for example. Such measurement may be performed using a device called TriIRIS. Pupillometry at least includes measurement of pupillary response (light reflex) to a visible light stimulus from a visual target, and measurement of pupillary changes at the time of looking at a moving visual target. Specifically, pupillary changes occur due to pupillary near reflex, and a pupil constricts when the visual target moves close. Furthermore, specifically, in the near point distance measurement, a measurement target person presses a switch at hand when a visual target approaching at a constant refraction speed becomes blurry during observation, and a visual target position at the time is recorded as a near point distance. Such measurements are measurement of a state of an eye (pupil) and measurement of a near point distance in response to pressing a switch at a time point of blurring, and the main objectives are measurement of the near point distance.

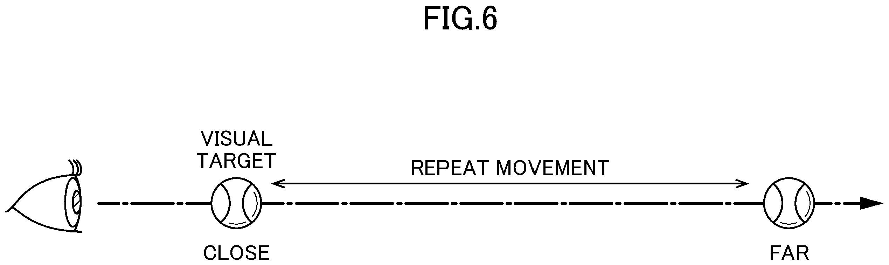

The three-dimensional cognitive ability also especially includes an ability of accurately following a moving object with eyes, correctly recognizing a position thereof, and making an appropriate response thereto. To measure a function of following a moving object with eyes, methods of measuring pupil modulation and convergence reflex are considered particularly effective. These measurement methods will be described later. Figure 6 is a diagram showing a concept of a visual target used in measuring a function of an eye. A visual target is moved in front of eyes of a measurement target person, in a direction of moving away and a direction of moving closer, and the measurement target person is made to visually recognize the visual target. Preferably, movement is repeated, and the eyes of the measurement target person are checked each time.

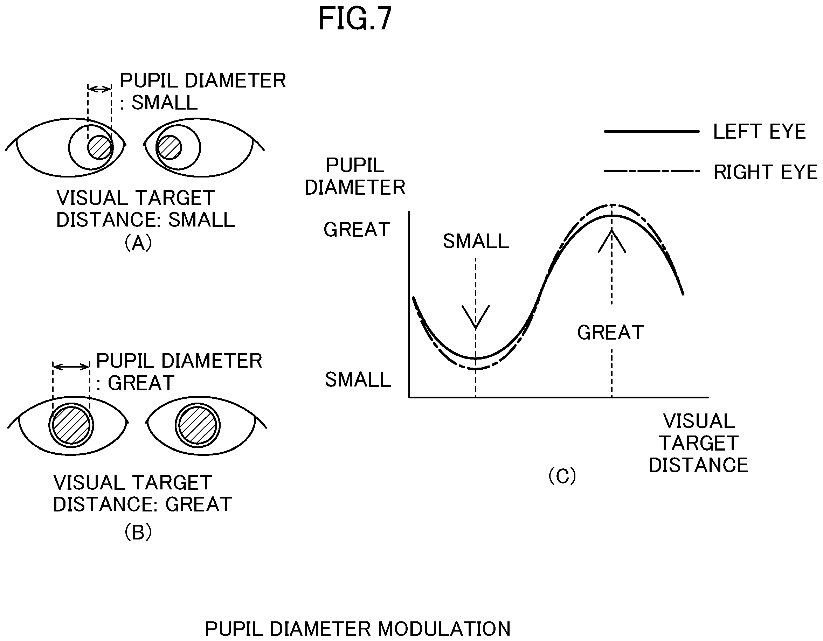

Figure 7 shows a relationship between a visual target distance (a distance between the eyes of the measurement target person and the visual target) and pupil diameters based on pupillary near reflex. Figure 7(A) shows that the pupil diameters decrease when the visual target distance is small, and Figure 7(B) shows that the pupil diameters increase when the visual target distance is great. Figure 7(C) shows a graph that takes the visual target distance as a horizontal axis, and the pupil diameter as a vertical axis. In the graph, a solid line indicates a left eye, and a dash-dotted line indicates a right eye. The graph shows that the pupil diameters decrease when the visual target distance is small, and that the pupil diameters increase when the visual target distance is great. It is also shown that the pupil diameter is approximately the same for the right eye and the left eye regardless of the visual target distance.

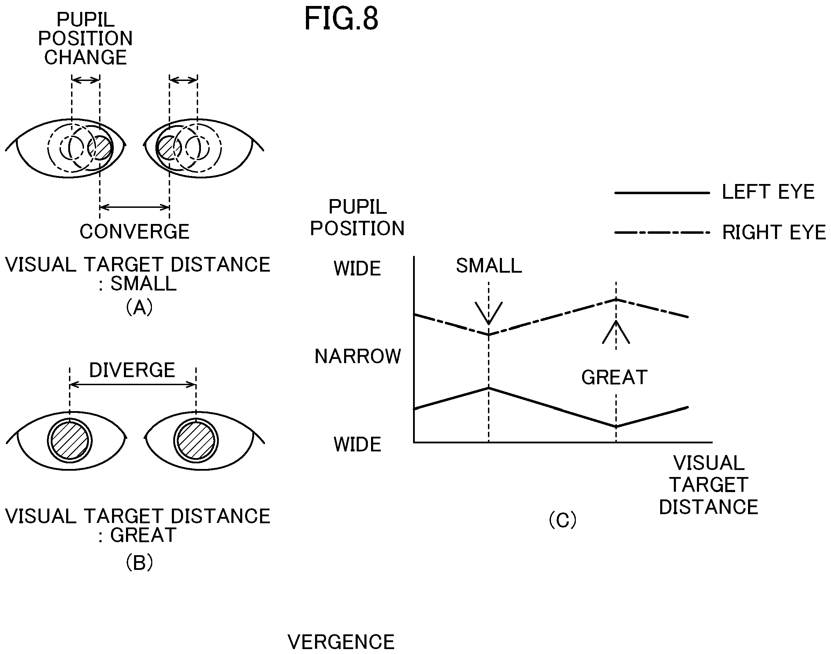

Figure 8 shows a relationship between the visual target distance and pupil positions (vergence). Figure 8(A) shows that the left and right eyes are in a converged state of being rotated inward when the visual target distance is small, and Figure 8(B) shows that the left and right eyes are in a parallel state and are in a diverged state when the visual target distance is great. Figure 8(C) shows a graph that takes the visual target distance as a horizontal axis, and the pupil position as a vertical axis. In the graph, a solid line indicates the left eye, and a dash-dotted line indicates the right eye. The graph shows that the left and right eyes are in the converged state with a distance between the pupils of the left and right eyes being reduced when the visual target distance is small, and that the left and right eyes are in the diverged state with the distance between the pupils of the left and right eyes being increased when the visual target distance is great.

When the visual target the measurement target person is looking at is moved such that distance/depth is changed, responses such as a change in the pupil diameter and the vergence as mentioned above occur. However, if visual cognitive function of the measurement target person is reduced, such responses are reduced. Accordingly, the visual cognitive function may be measured by measuring the change in the pupil diameter and the vergence of the measurement target person when the distance/depth of the visual target is changed. However, this requires a large-scale apparatus for changing the distance/depth of the visual target. Furthermore, TriIRIS performs measurement based on the pressing of a switch at a time point of blurring; however, a response of pressing a switch is a passive response that is taken when a visual target that is a visual cognitive target object that moves one-dimensionally independently of an operation by the measurement target person moves to a predetermined position, and is not a response that is based on an active motion of the measurement target person that is taken in relation to the moving visual target (a response that requires an active operation where a target position is dynamically determined by the position of the visual cognitive target object, such as bringing a hand or the like close to the visual target), and may thus possibly obtain a good result by chance, or a good result is possibly obtained by the measurement target person telling a lie. In this manner, measurement based on a passive response of simply pressing a switch in good timing with a moving object that is being visually recognized has a problem of accuracy.

Now, as virtual reality (VR) technology develops, its application fields are expanding. When a user wears a virtual reality headset for providing virtual reality, a virtual object is displayed in a line-of-sight direction, and a feeling of as if being present together may be obtained. With the virtual reality headset, electronic displays are embedded inside a housing that is shaped like goggles and an image of an object that is present in the line-of-sight direction is displayed thereon, and a user visually recognizes the image through eyepieces. The electronic displays are separately provided for left and right eyes, and an appropriate sense of distance/depth is provided to the user by changing a display position according to a position of the displayed object in a distance/depth direction. That is, in relation to a nearby object, corresponding images to be displayed on the left and right electronic displays are displayed close to a center, thus causing the eyes of the user to converge and to recognize the object to be at a close position. When measuring a function of the eyes, it is conceivable to use such a virtual reality headset to simulate change in the position of the visual target, but there is no such technique.

Now, as a system for evaluating a cognitive function using measurement of vision, there is a system that uses a portable touchscreen personal computing device (Patent Literature 1). With this technique, cognitive assessment of an individual is performed based on a response speed to a cognitive assessment stimulus that is displayed. In a test, when a letter is displayed, a response is made by the pressing of a button, and measurement is performed. However, with this technique, a displayed object is moved, but there is no mention of a size of distance/depth of the object. Furthermore, with this technique, measurement results vary depending on various attributes such as a level of concentration of a measurement target person or a level of skill in an operation method.

Furthermore, there is a system for providing a video game that performs the mapping of a peripheral field of view of a subject, including a test performed by the subject to find a visual stimulus that is presented for a short period of time (Patent Literature 2). With this technique, a target is displayed on a display, and visual field defect caused by glaucoma is measured based on a response of the user to the target. This technique measures a distance between the subject and a monitor, but makes no mention of a size of distance/depth to the target that is displayed.

### Citation List

### Patent Literature

Patent Literature 1: Japanese Patent No. 6000968
Patent Literature 2: Japanese Translation of PCT International Application Publication No. 2015-502238

### Summary of Invention

### Technical Problem

As described above, it is difficult to objectively evaluate the cognitive function, and enabling objective evaluation by quantifying the three-dimensional cognitive ability related to the cognitive function is very useful, but there is no such technique. Moreover, with measurement based on a passive response of pressing a switch when a moving visual target reaches a predetermined position, good results may be obtained by chance or a false statement, and there is a problem of accuracy. Moreover, to check a visual cognitive function to evaluate the three-dimensional cognitive ability, a distance/depth of a visual target that is visually recognized by a measurement target person needs to be changed, and this requires a large-scale apparatus for physically moving the visual target. There is a computing device that is an apparatus for checking the visual cognitive function, but this is not able to change a sense of distance/depth. Accordingly, there is a demand for a method of accurately quantifying the three-dimensional cognitive ability. That is, there is a demand for a small apparatus that is capable of evaluating the three-dimensional cognitive abilities including the visual cognitive function, by making a measurement target person visually recognize an object whose distance/depth is changed and by measuring a response thereto.

The present invention has been made in view of the above problems, and is aimed at providing a small apparatus that is capable of providing a method of quantifying the three-dimensional cognitive ability, and of evaluating the three-dimensional cognitive ability.

### Solution to Problem

A three-dimensional cognitive ability evaluation system according to a mode of the present invention is for evaluating a three-dimensional cognitive ability based on a response of a measurement target person to a moving object, and includes an object position acquisition unit configured to acquire a position of the moving object, the position enabling identification of a distance between the moving object and the measurement target person; a response input unit configured to receive an input of an active response of the measurement target person taken in response to a position of the object that is recognized by the measurement target person; and a three-dimensional cognitive ability determination unit configured to evaluate the three-dimensional cognitive ability of the measurement target person by determining whether the position of the object that is acquired and the response that is input correctly match.

In the present invention, the three-dimensional cognitive ability determination unit may evaluate the three-dimensional cognitive ability of the measurement target person based on a positional correspondence relationship between positions of the object that are acquired and positions that are identified based on the response, within a predetermined range of time. In the present invention, the positional correspondence relationship may include at least one of a smallest distance between the position of the object and the position that is identified based on the response, an average distance between the position of the object and the position that is identified based on the response, and a difference between a greatest distance and the smallest distance between the position of the object and the position that is identified based on the response.

In the present invention, the moving object may be an operation target object that is moved from a departure position toward a target position by an operation by the measurement target person, the response input unit may receive a position of the operation target object as the input of the response, and the three-dimensional cognitive ability determination unit may evaluate the three-dimensional cognitive ability of the measurement target person based on a difference between the position of the operation target object and the target position.

In the present invention, there may be further included an eyeball state sensing unit configured to sense line-of-sight directions of both eyes of the measurement target person; and a visual recognition determination unit configured to determine whether the object is spatially recognized visually by the measurement target person, by determining whether the line-of-sight directions correctly match the position of the object that is moving, where the three-dimensional cognitive ability determination unit may evaluate the three-dimensional cognitive ability of the measurement target person by determining, in a case where visual and spatial recognition of the object by the measurement target person is determined by the visual recognition determination unit, whether the response that is input correctly matches the position of the object that is acquired.

In the present invention, in a case where the line-of-sight directions of both eyes are each determined to coincide with (corresponding to) the position of the moving object, for a predetermined period of time or longer, the visual recognition determination unit may determine that the object is visually recognized by the measurement target person.

In the present invention, the eyeball state sensing unit may further sense pupil diameters of both eyes of the measurement target person, and in a case of further determining that the pupil diameters of both eyes are gradually reduced as the position of the object moves closer to predetermined point of view, the visual recognition determination unit may determine that the object is spatially recognized visually by the measurement target person.

In the present invention, the moving object may be provided in virtual reality, there may be further included a virtual reality headset including an electronic display for displaying a moving image in the virtual reality, and a moving object display unit configured to cause the electronic display to display a moving image in which the object seen from a predetermined point of view in the virtual reality moves from a movement start position to a movement end position along a predetermined movement route in a direction of approaching the predetermined point of view, the object position acquisition unit may acquire the position of the object in the virtual reality displayed by the movement object display unit, and the three-dimensional cognitive ability determination unit may evaluate the three-dimensional cognitive ability of the measurement target person by determining whether the response that is input correctly matches the position of the object that is acquired.

In the present invention, the response input unit may continuously identify a position of a predetermined part of a body of the measurement target person based on a signal from a sensor attached to the predetermined part of the body, where the position of the predetermined part of the body is input as the response, the movement object display unit may further cause an image of at least a part of the predetermined part of the body of the measurement target person to be displayed in the virtual reality on the electronic display, based on the position of the predetermined part of the body that is identified, and the three-dimensional cognitive ability determination unit may determine the correct matching of the response in a case where a distance between a predetermined portion related to the predetermined part of the body and the object falls to or below a predetermined distance in a case where spatial recognition of the object by the measurement target person is determined by the visual recognition determination unit.

In the present invention, the three-dimensional cognitive ability determination unit may acquire three response parameters including a visual recognition start time from a movement start time of the object to when spatial recognition of the object by the measurement target person is determined, a smallest distance between a predetermined portion related to a predetermined part of the body and the object, and a response time from the movement start time of the object to when a distance between the predetermined portion related to the predetermined part of the body and the object reaches the smallest distance, and may evaluate the three-dimensional cognitive ability of the measurement target person based on the response parameters.

In the present invention, the three-dimensional cognitive ability determination unit may calculate scores based on numerical values of the response parameters, and may evaluate the three-dimensional cognitive ability of the measurement target person based on a total of the scores that are multiplied by respective predetermined weights.

In the present invention, movement of the object by the moving object display unit, determination by the visual recognition determination unit of whether the object is spatially recognized visually by the measurement target person, and evaluation of the three-dimensional cognitive ability by the three-dimensional cognitive ability determination unit may be repeated a predetermined number of times of measurement, and the three-dimensional cognitive ability determination unit may output a number of times when the response is determined to correctly match the position of the object.

The present invention is also implemented as an apparatus that includes, in one housing, structures for evaluating a three-dimensional cognitive ability based on a response of a measurement target person to a moving object. The present invention is also implemented as a program for causing a computer to realize a three-dimensional cognitive ability evaluation system for evaluating a three-dimensional cognitive ability based on a response of a measurement target person to a moving object, by being executed by the computer, and a computer-readable recording medium storing the program. The present invention is also implemented as a method including steps for evaluating a three-dimensional cognitive ability based on a response of a measurement target person to a moving object.

### Advantageous Effects of Invention

The present invention evaluates a three-dimensional cognitive ability of a measurement target person through acquisition of a position of an object, the position enabling identification of a distance to the measurement target person, reception of an input of an active response of the measurement target person taken in response to a position of the object recognized by the measurement target person, and determination of whether the position of the object that is acquired and the response that is input correctly match. Accordingly, whether a correctly matched response is taken in response to a position of an object that needs to be spatially recognized through a visual way (visually) may be checked based on the position of the object and the response, and an advantageous effect may be obtained according to which a three-dimensional cognitive ability related to a cognitive function may be quantified and objectively evaluated. Furthermore, in the case where the present invention is configured such that a moving object is taken as an operation target object that is moved by an operation by the measurement target person, from a departure position to a target position, a position of the operation target object is received as an input of a response, and the three-dimensional cognitive ability of the measurement target person is evaluated based on a difference between the position of the operation target object and the target position, an advantageous effect may be obtained according to which the three-dimensional cognitive ability related to a cognitive function may be quantified and objectively evaluated without making the measurement target person bored with a measurement test, by using operation of an operation target object such as a drone that is interesting and that brings a sense of accomplishment.

Furthermore, in the present invention, a measurement result is obtained based on an active response requiring an active operation, where a target position is dynamically determined by a position of a visual cognitive target object, and thus, it is possible to eliminate a possibility that a good measurement result is obtained by chance by a passive operation of pressing a switch in relation to a target that moves one-dimensionally, and an advantageous effect that objectivity and accuracy of measurement are increased may be obtained. Furthermore, in the present invention, measurement is performed by a simple and interesting measurement test where a measurement result is not dependent on various attributes (a level of concentration, a level of skill in operation method, a tendency to lie, etc.) of the measurement target person, and thus, the level of concentration may be measured based on reliability of visual recognition, and an advantageous effect that accurate measurement may be performed by eliminating lies may be obtained. Moreover, in the present invention, the moving object may be provided in virtual reality, and the present invention in this case uses a virtual reality headset that includes an electronic display for displaying a moving image in virtual reality, causes a moving image when an object seen from a predetermined point of view is moved in virtual reality from a movement start position to a movement end position in a direction of approaching the predetermined point of view, along a predetermined movement route, to be displayed on the electronic display, receives input of an active response taken in response to a position of the object recognized by the measurement target person, and determines whether the position of the object that is acquired and the response that is input correctly match, and thus, a large-scale apparatus is not necessary, and movement of an object may be accurately simulated while also reproducing a sense of distance/depth, and a measurement test may be performed based on the simulation by using a small apparatus, and an advantageous effect that the three-dimensional cognitive ability may be simply and reliably quantified and objectively evaluated may thus be obtained.

### Brief Description of Drawings

[Figure 1] Figure 1 is a diagram showing a schematic external appearance of a three-dimensional cognitive ability evaluation system 100 according to an embodiment of the present invention.
[Figure 2] Figure 2 is a block diagram showing a configuration of the three-dimensional cognitive ability evaluation system 100 according to the embodiment of the present invention.
[Figure 3] Figure 3 is a functional block diagram showing a functional configuration of the three-dimensional cognitive ability evaluation system 100 according to the embodiment of the present invention.
[Figure 4] Figure 4 is an operation flow diagram of the three-dimensional cognitive ability evaluation system 100 according to the embodiment of the present invention.
[Figure 5] Figure 5 is a diagram describing a visual recognition start time and a response time.
[Figure 6] Figure 6 is a diagram describing a response of eyes to a moving object (movement of a visual target).
[Figure 7] Figure 7 is a diagram describing a response of the eyes to the moving object (pupil diameters).
[Figure 8] Figure 8 is a diagram describing a response of the eyes to the moving object (vergence).
[Figure 9] Figure 9 is an image view of use of the three-dimensional cognitive ability evaluation system 100.
[Figure 10] Figure 10 is a diagram showing an example of a display screen in a measurement test for measurement of a three-dimensional cognitive ability based on the catching of a ball that is pitched.
[Figure 11] Figure 11 is a diagram showing an example of the display screen in the measurement test for measurement of the three-dimensional cognitive ability based on the catching of a ball that is pitched.
[Figure 12] Figure 12 is a diagram showing an example of a display screen in a measurement test for measurement of the three-dimensional cognitive ability based on the hitting of a pitched ball with a bat.
[Figure 13] Figure 13 is an example of a table of expected values of response parameters based on age.
[Figure 14] Figure 14 is an example of a table of expected values of response parameters based on rank of skill level.
[Figure 15] Figure 15 is a diagram showing an example of a display screen in a measurement test for measurement of the three-dimensional cognitive ability based on squash.
[Figure 16] Figure 16 is a diagram showing an example of a measurement result of the measurement test for measurement of the three-dimensional cognitive ability based on squash.
[Figure 17] Figure 17 is a diagram showing an example of a display screen in a measurement test for measurement of the three-dimensional cognitive ability based on driving simulation.
[Figure 18] Figure 18 is a diagram showing an example of a measurement result of the measurement test for measurement of the three-dimensional cognitive ability based on driving simulation.
[Figure 19] Figure 19 is an image view of a measurement test for measurement of the three-dimensional cognitive ability based on a drone landing operation.
[Figure 20] Figure 20 is a diagram showing an example of a measurement result of the measurement test based on the drone landing operation.

### Description of Embodiment

### (Configuration of Three-Dimensional Cognitive Ability Evaluation System 100)

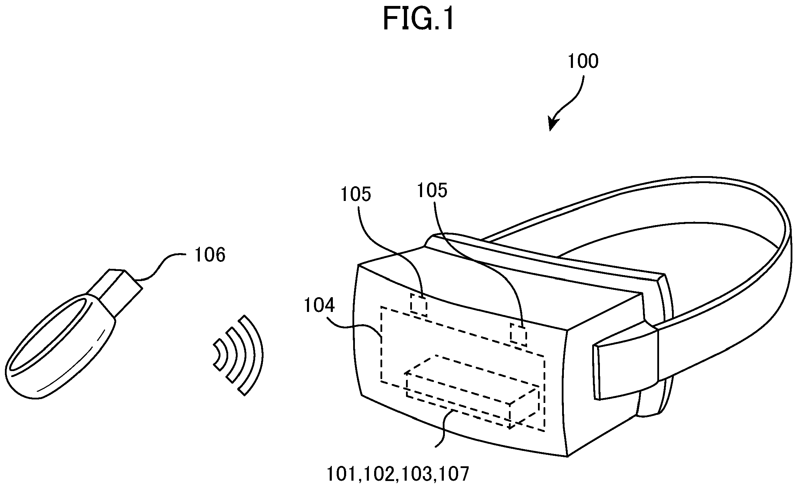

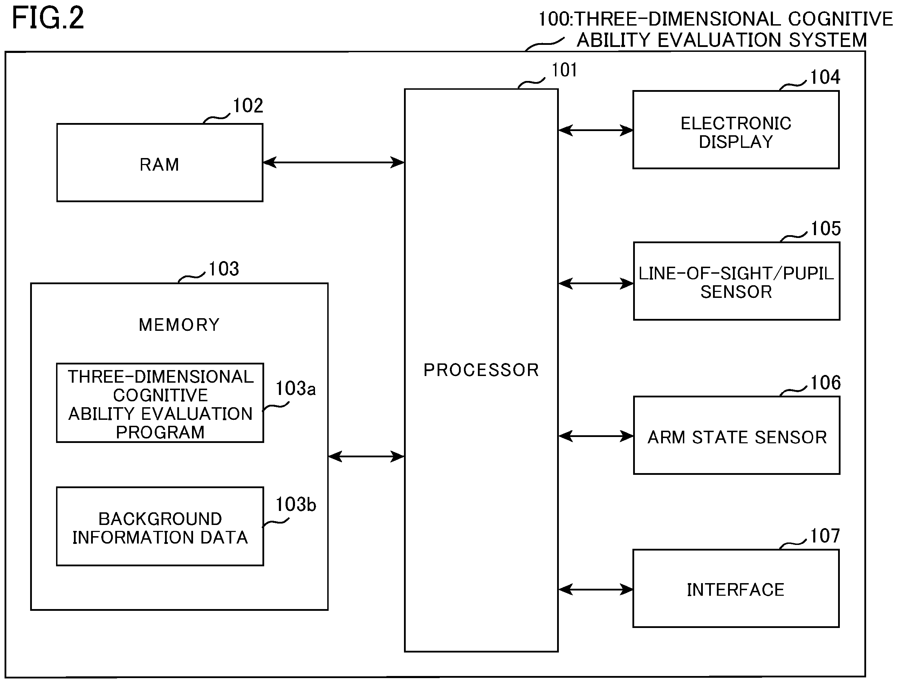

Hereinafter, a three-dimensional cognitive ability evaluation system 100 according to an embodiment of the present invention will be described with reference to the drawings. Figure 1 shows a schematic external appearance of the three-dimensional cognitive ability evaluation system 100. Components shown by dashed lines in Figure 1 are components that are present inside a main body of the three-dimensional cognitive ability evaluation system 100 and that cannot be seen from outside. Details of these components will be given later with reference to Figure 2. The three-dimensional cognitive ability evaluation system 100 is a system for evaluating a three-dimensional cognitive ability of a measurement target person by making the measurement target person visually recognize a moving object, and by evaluating a resulting response of the measurement target person. A response in the present invention is to recognize a distance/depth of an object and to respond to the same. Furthermore, a measurement target person in the present invention is a person whose three-dimensional cognitive ability is measured.

The three-dimensional cognitive ability evaluation system 100 typically takes a form of a virtual reality headset that is a head-mounted display (goggles) provided with electronic displays for displaying a moving image representing three-dimensional virtual reality. A band for mounting such as a rubber band is typically attached to the three-dimensional cognitive ability evaluation system 100. A user attaches the three-dimensional cognitive ability evaluation system 100 around eyes by placing the three-dimensional cognitive ability evaluation system 100 to cover the eyes and wrapping the rubber band around head.

Figure 2 is a block diagram showing a configuration of the three-dimensional cognitive ability evaluation system 100. The three-dimensional cognitive ability evaluation system 100 includes a processor 101, a RAM 102, a memory 103, an electronic display 104, a line-of-sight/pupil sensor 105, an arm state sensor 106, and an interface 107. The processor 101 is a processing circuit for performing various functions for controlling operation of the three-dimensional cognitive ability evaluation system 100, and is typically a CPU for operating an information appliance such as a computer. The RAM 102 is a temporary memory, and is used as a work area at a time of operation of the processor 101, a storage area for temporary data, and the like. The memory 103 is typically a non-volatile memory such as a flash ROM, and stores computer programs and data that is referred to at the time of execution of the computer programs. The memory 103 stores a three-dimensional cognitive ability evaluation program 103a as a computer program. It is noted that at a time of execution of a computer program, an operating system (OS) is usually used, but a function of the OS is assumed to be included in a function of execution of the computer program by the processor 101, and description thereof is omitted. Characteristic functions of the three-dimensional cognitive ability evaluation system 100 according to the present invention are implemented by execution of the computer programs by the processor 101, by which execution modules corresponding to the functions are formed. Modules for implementing various functions related to evaluation of a three-dimensional cognitive ability are formed by the processor 101 reading out, and executing using the work area in the RAM 102, the three-dimensional cognitive ability evaluation program 103a stored in the memory 103, and operation for implementing the functions is thus performed.

The memory 103 stores background information data 103b as data that is referred to at the time of execution of the three-dimensional cognitive ability evaluation program 103a. The background information data 103b is typically data of an expected value indicating a general test result, and is data that is referred to at the time of evaluating a response of a measurement target person through comparison with the expected value.

It is noted that not all the functions to be implemented by the three-dimensional cognitive ability evaluation program 103a have to be performed by a processor in a housing of the head-mounted display. For example, a part of the three-dimensional cognitive ability evaluation program 103a, the background information data 103b and the like may be stored in an external smartphone or the like to be executed by a processor of the smartphone. In this case, a function of a part of the three-dimensional cognitive ability evaluation program 103a that is performed by the processor 101 in the housing of the head-mounted display and a function of a part of the three-dimensional cognitive ability evaluation program 103a that is performed by the external smartphone or the like communicate with each other as appropriate to thereby implement the function of three-dimensional cognitive ability evaluation as a whole.

The electronic display 104 is a flat panel display such as a liquid crystal display (LCD) or an organic EL display, and displays a moving image of a moving object in virtual reality, to a user wearing the three-dimensional cognitive ability evaluation system 100 around the eyes, through an eyepiece disposed on a measurement target person side. When data of the moving image to be displayed is transferred to a data buffer area in the electronic display 104, the electronic display 104 reads data of an image from the data buffer area and displays the corresponding moving image. The electronic display 104 is separately provided for a right eye and a left eye, and is viewed by the user through the eyepiece. In the case where a position of the object to be displayed is infinite, the object is displayed at a same position on the electronic displays 104 for the right eye and the left eye and no parallax is generated between the left and right eyes, and the left and right eyes are placed in a diverged state and a sense of being present at an infinite distance is felt by the user. As the position of the object that is displayed comes closer to the user, it is displayed more inward on the electronic displays 104 for the right eye and the left eye, and parallax is generated between the left and right eyes, and the left and right eyes are placed in a converged state, and a sense of being close is felt by the user.

The line-of-sight/pupil sensor 105 is a sensor that is disposed facing the measurement target person, above the electronic display 104, for example, and that detects a line-of-sight direction of the left/right eye and a size of a pupil, and is a component that functions as an eyeball state sensing unit. The line-of-sight/pupil sensor 105 acquires an image of the left/right eye with image acquisition means such as a camera, and determines, and outputs, the line-of-sight direction and the size of the pupil by identifying a position of the pupil in the image and the size of the pupil. As the camera, a visible light camera or an infrared camera may be used. First, to determine whether an object is visually recognized, the line-of-sight direction is important data. That an object is visually recognized may be confirmed by checking that each line of sight (a normal line of a center portion of the pupil) of the left and right eyes surely passes through the object. At this time, if a nearby object is visually recognized, a converged state is reached due to the lines of sight of the left and right eyes moving inward due to parallax. Furthermore, to determine that an approaching object is being continuously visually recognized, a pupil diameter may be used in addition. In the case where an approaching object is being visually continuously recognized, the pupil diameter is gradually reduced due to pupillary near reflex, and by detecting such a state, whether visual recognition is successful or not may be checked. The arm state sensor 106 is a sensor that is attached to an arm of the measurement target person to detect a state of the arm, such as a position or a direction, and is a sensor for detecting motion, position or direction, such as a gyrosensor, an accelerometer or an azimuth sensor. The arm state sensor 106 is connected to the processor 101 by wired or wireless connection. It is noted that the arm state sensor 106 may be replaced with a sensor that is attached to a predetermined part of a body other than the arm to detect a state of the predetermined part of the body, such as a position or a direction. The interface 107 is a user interface for allowing a user to input information such as an operation instruction, and for outputting information indicating an operation state to the user, and includes input means such as operation buttons, a touch panel and an answer selection button, and output means such as an LED. Furthermore, in the case where a part of the three-dimensional cognitive ability evaluation program 103a is executed by an external smartphone or the like, the interface 107 also includes wireless communication means such as Wi-Fi (registered trademark) or Bluetooth (registered trademark) to communicate with the external smartphone or the like.

### (Functional Blocks of Three-Dimensional Cognitive Ability Evaluation System 100)

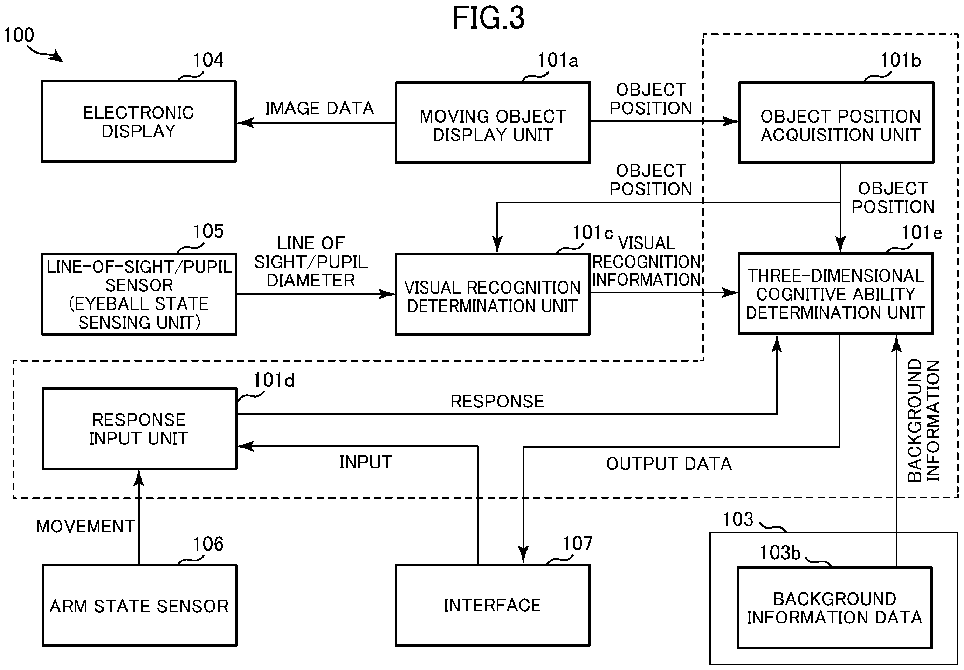

Next, a functional configuration of the three-dimensional cognitive ability evaluation system 100 will be described. Figure 3 is a functional block diagram showing the functional configuration of the three-dimensional cognitive ability evaluation system 100. With the three-dimensional cognitive ability evaluation system 100, modules forming functional blocks including a moving object display unit 101a, an object position acquisition unit 101b, a visual recognition determination unit 101c, a response input unit 101d, and a three-dimensional cognitive ability determination unit 101e are implemented by execution, by the processor 101, of the three-dimensional cognitive ability evaluation program 103a stored in the memory 103. Accordingly, Figure 3 shows, instead of the processor 101 and the three-dimensional cognitive ability evaluation program 103a in Figure 2, functional blocks that are implemented by those mentioned above. In the following, the functional blocks will be described.

The moving object display unit 101a is a functional block for causing the electronic display to display a moving image in which an object seen from a predetermined point of view in virtual reality is moved from a movement start position to a movement end position along a predetermined movement route in a direction of approaching the predetermined point of view. The moving object display unit 101a generates a moving image, for measurement of a visual cognitive function, formed from continuous images forming a video of a moving object, and transmits image data for displaying the same to the electronic display 104 for display. For example, in the case of measurement of a response when a measurement target person sees a pitched ball, the moving object display unit 101a generates an image of a background, and also, generates the predetermined movement route for the ball as the moving object, from a pitching position of a pitching person as the movement start position to a catching position by a catching person as the movement end position and causes information about a position of the ball to move along the predetermined movement route, and continuously generates images of the ball seen from a point of view of each of the left and right eyes of the catching person by three-dimensional rendering and generates image data by superimposing the generated image upon the image of the background, and transfers the same to the data buffer area of the electronic display 104 as data representing the moving image. The image data is data for each of the electronic displays 104 for the right eye and the left eye, and parallax is generated in relation to the positions of the object in the images for the right eye and the left eye according to the position (a distance from the user) of the moving object. Accordingly, the measurement target person looking at the moving image on the electronic displays 104 looks at the ball at a realistic distance/depth. The moving object display unit 101a transmits the position of the ball as an object position to the object position acquisition unit 101b so that determination using the object position is performed.

The object position acquisition unit 101b acquires information about the position of the object that is generated by the moving object display unit 101a and that is used in the simulation, and transmits the information to the visual recognition determination unit 101c and the three-dimensional cognitive ability determination unit 101e. The information about the position of the object is information that enables identification of at least a distance between the object and the measurement target person, and is typically three-dimensional positional information. A sense of distance between an object and a measurement target person is a sense that is necessary at a time of taking a predetermined response to a target whose position is dynamically determined based on the position of a moving object which is a visual cognitive target object, as in the case of catching an approaching object or maintaining a constant distance to an object in front. In the case of using a specific three-dimensional position (three-dimensional coordinates) of the measurement target person, a three-dimensional position of the object may be used as the information about the position of the object, and in this case, the distance may be determined from coordinates representing the three-dimensional positions of the two by using a distance formula. Furthermore, in the case where specific three-dimensional positions of the measurement target person and the object are not used as the information about the position of the object, information about only the distance to the object may be used. In the case where information about the three-dimensional position of the object is used as the information about the position of the object, a size of distance/depth along the line of sight of the measurement target person and the line-of-sight direction may be identified. The object position acquisition unit 101b typically extracts the position of the object generated by the moving object display unit 101a for display, to be used by the visual recognition determination unit 101c and the three-dimensional cognitive ability determination unit 101e, and is a functional block that is implemented by execution of a routine for acquiring the position of the object for functional blocks that need the position of the object, such as the visual recognition determination unit 101c and the three-dimensional cognitive ability determination unit 101e. It is noted that in the case where an actual position of the object is used in the measurement test as in the fourth modification described later, instead of the position of the object being generated by the moving object display unit 101a, the object position acquisition unit 101b acquires the position of the object from a sensor or the like.

The visual recognition determination unit 101c is a functional block for determining whether an object that is moving is spatially recognized visually by the measurement target person, by determining whether the line-of-sight direction correctly matches the position of the object. The visual recognition determination unit 101c receives data about the line-of-sight directions of the left and right eyes of the measurement target person sensed by the line-of-sight/pupil sensors 105 that each function as the eyeball state sensing unit, and determines whether the line-of-sight directions of the left and right eyes match the object position transmitted from the moving object display unit 101a and whether the measurement target person is following the moving object with eyes, and thus determines whether the object is spatially recognized by the measurement target person. The visual recognition determination unit 101c may further receive data about the pupil diameters of the left and right eyes of the measurement target person sensed by the line-of-sight/pupil sensors 105, and may operate to determine that the object is spatially recognized by the measurement target person, in a case where it is further determined that the pupil diameters of the two eyes are gradually reduced while the position of the object approaches a predetermined point of view and the size of distance/depth is reduced (in the case where there is occurrence of pupillary near reflex due to the size of distance/depth being reduced).

The response input unit 101d is a functional block for receiving input of an active response of the measurement target person taken in response to the three-dimensional position of the object recognized by the measurement target person. A response of the measurement target person looking at the object that is moving is input to the response input unit 101d based on movement information about an arm of the measurement target person from the arm state sensor 106 or input information of an operation on a button or a touch panel performed by the measurement target person via the interface 107. It is noted that an active response means an operation that is performed on a target whose position is dynamically determined based on the position of the moving object. An active response is an active operation for achieving a predetermined objective, such as bringing a moving object close to a predetermined location (in this case, a difference between the position of the moving object and a position of the predetermined location is dynamically determined based on the position of the object, and it is aimed to reduce the difference), bringing a predetermined part of a body or the like close to a moving object (in this case, a difference between the position of the moving object and a position of the predetermined part of the body is dynamically determined, and it is aimed to reduce the difference), or maintaining a constant distance between a moving object and oneself (in this case, a difference between the position of the moving object and a position of oneself is dynamically determined based on the position of the object, and it is aimed to keep the difference at a constant value). In relation to the active response, visual recognition of a visual cognitive target object by the measurement target person, and accurate response based on three-dimensional recognition of the same greatly affect the result. That is, if the visual cognitive target object is not accurately visually recognized by the measurement target person, it cannot be accurately three-dimensionally recognized in a three-dimensional space. If the visual cognitive target object is not accurately three-dimensionally recognized, an accurate response cannot be taken in relation to a target whose position is dynamically determined by the visual cognitive target object. Accordingly, by checking the accuracy of an active response, the three-dimensional cognitive ability can be accurately evaluated. In contrast, a passive response is a response that is taken when a visual cognitive target object moving one-dimensionally irrespective of an operation by the measurement target person is recognized to have reached a predetermined position, and is a response that does not require much three-dimensional recognition by the measurement target person. Accordingly, with the passive response, a measurement result may often be better than an actual ability by chance. Accordingly, the measurement result is not an accurate evaluation of the three-dimensional cognitive ability.

The three-dimensional cognitive ability determination unit 101e is a functional block for evaluating the three-dimensional cognitive ability of the measurement target person by determining whether the position of the object and the response of the measurement target person correctly match. The three-dimensional cognitive ability determination unit 101e checks whether the response correctly matches the position of the object, by checking whether the response of the measurement target person from the response input unit 101d matches the position of the object. That is, in the case where a target response is to bring a moving object close to a predetermined location, it is checked that a difference between a position of the moving object and a position of the predetermined location is reduced to or below a predetermined value (that the positions substantially coincide with each other), and in the case where the target response is to bring a predetermined part of the body or the like close to the moving object, it is checked that a difference between the position of the moving object and a position of the predetermined part of the body is reduced to or below a predetermined value (that the positions substantially coincide with each other), and in the case where the target response is to maintain a constant distance between the moving object and oneself, it is checked that a difference between the position of the moving object and a position of oneself is close to a constant value (that the difference between the positions is substantially constant). The three-dimensional cognitive ability determination unit 101e may also determine whether the response correctly matches the position of the object, with determination by a recognition determination unit that object is visually and spatially recognized by the measurement target person as an additional condition. Moreover, deep learning or the like may be used in determination of the three-dimensional cognitive ability.

Of the functional blocks described above, the object position acquisition unit 101b, the response input unit 101d, and the three-dimensional cognitive ability determination unit 101e are functional blocks that are particularly necessary for determination of the three-dimensional cognitive ability. In Figure 3, these functional blocks are surrounded by a dashed line. In the fourth modification described later, the functional blocks of the object position acquisition unit 101b, the response input unit 101d and the three-dimensional cognitive ability determination unit 101e form a main part of the system.

### (Operation of Three-Dimensional Cognitive Ability Evaluation System 100)

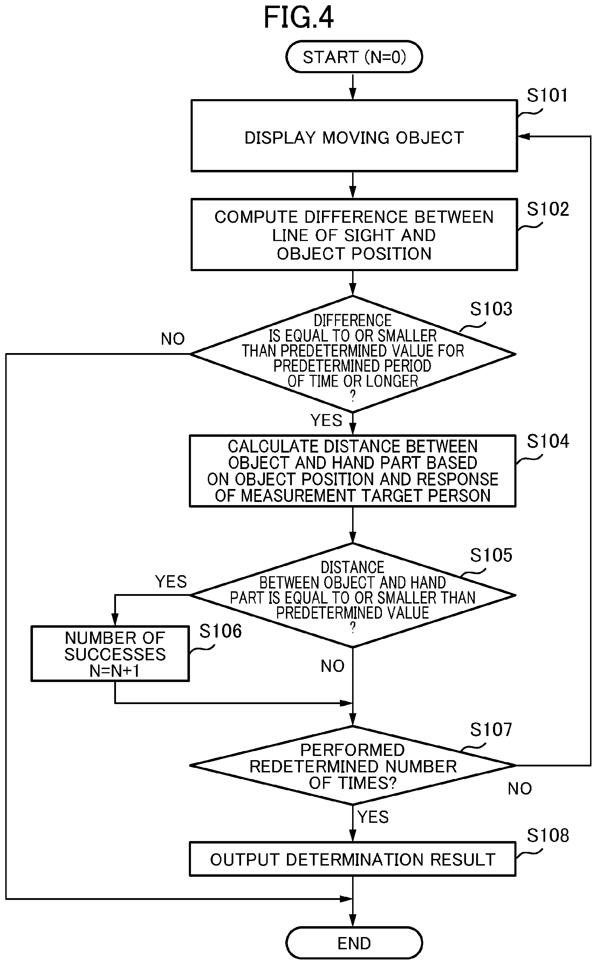

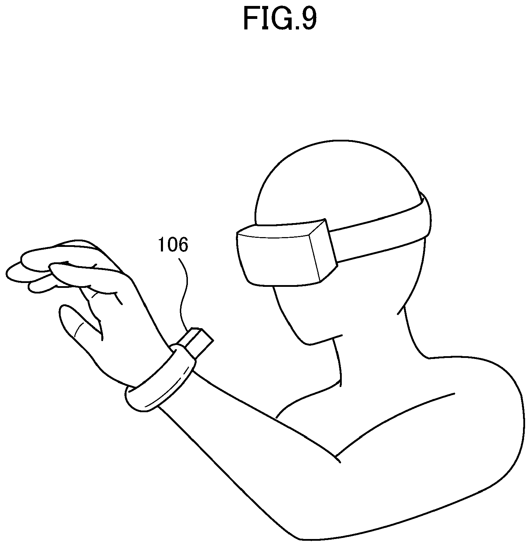

Next, an operation of the three-dimensional cognitive ability evaluation system 100 will be described with reference to an operation flow shown in Figure 4. To determine the three-dimensional cognitive ability, the three-dimensional cognitive ability evaluation system 100 evaluates whether an appropriate response corresponding to a position of a moving object is taken, by performing a measurement test using simulation. As the measurement test, a test of catching a moving object is typically cited, for example. In this example, as the measurement test performed in the form of simulation to determine the three-dimensional cognitive ability, a test of success/failure or degrees of skill (dexterity) in the catching of a pitched ball is performed. An active response of the measurement target person is to bring a hand part that is a predetermined part of a body or a catching tool held by the hand part close to a ball that is the moving object. The measurement target person is to bring the hand part or the catching tool held by the hand part close to the position of the ball as the target. Accordingly, the ball is used as the moving object, a pitching position of a pitching person is used as the movement start position, a catching position of a catching person is used as the movement end position, a trajectory of the ball pitched by the pitching person is used as the predetermined movement route, and a point of view of the catching person is used as the predetermined point of view. More specifically, the pitching person is a pitcher in baseball, and the catching person is a catcher in baseball. First, the moving object display unit 101a causes the moving object to be displayed on the electronic displays 104 (step S101). That is, to measure the response of the measurement target person looking at a pitched ball, the moving object display unit 101a generates, in relation to the ball as the moving object, the predetermined movement route (that is, continuous positions of the ball) from the pitching position of the pitching person (pitcher) as the movement start position to the catching position of the catching person (catcher) as the movement end position, continuously generates, by three-dimensional rendering, images indicating the trajectory of the ball pitched by the pitching person, as seen from the point of view of the catching person, and superimposes the generated image upon the image of background (baseball field and batter's box) to generate image data, and transmits the same to the data buffer areas of the electronic displays 104. To generate images of a series of pitching movement, the moving object display unit 101a first takes a typical pitching position that is stored (or a position that is slightly and randomly changed, for example) as an initial position of the ball. Then, the moving object display unit 101a sets a speed and a movement route by adopting an appropriate pattern from a plurality of patterns of speed and movement route (or a pitching direction) that are stored in advance or by slightly and randomly shifting the route and the like from typical speed and movement route. The movement route of the ball and the speed during movement are desirably determined according to laws of physics such as gravity and air resistance, with a direction and speed at the time of pitching as initial values. It is noted that the movement end position of the movement route is the catching position when the ball is successfully caught. Then, the moving object display unit 101a moves the position of the ball from the pitching position to the catching position, along the predetermined movement route, generates, for the right eye and the left eye, series of images showing the ball at the positions, as seen from the point of view of the catching person, and transmits the same to the buffer areas of the electronic displays 104 to be displayed as a moving image. Preferably, the moving object display unit 101a further causes the hand part at a tip of an arm to be displayed, based on information about a position of the arm of the measurement target person acquired from the arm state sensor 106. An image of the hand part is not an image of a bare hand, but is of a catching tool (such as a mitt or a glove) of the catching person worn to cover the hand part. Figure 9 shows an image of use of the three-dimensional cognitive ability evaluation system 100. The measurement target person visually recognizes the ball displayed in virtual reality on the electronic displays 104, and moves the arm to catch the ball. Movement of the arm is detected by the arm state sensor 106, and the hand part displayed in virtual reality on the electronic displays 104 is moved accordingly. The measurement target person makes a movement of catching the ball by visually recognizing movement of the ball and moving the hand part that is displayed, to intersect the trajectory of the ball. It is noted that in the case where information about the position of the hand part is to be calculated by the three-dimensional cognitive ability determination unit 101e, the moving object display unit 101a may acquire the information about the position of the hand part from the three-dimensional cognitive ability determination unit 101e.

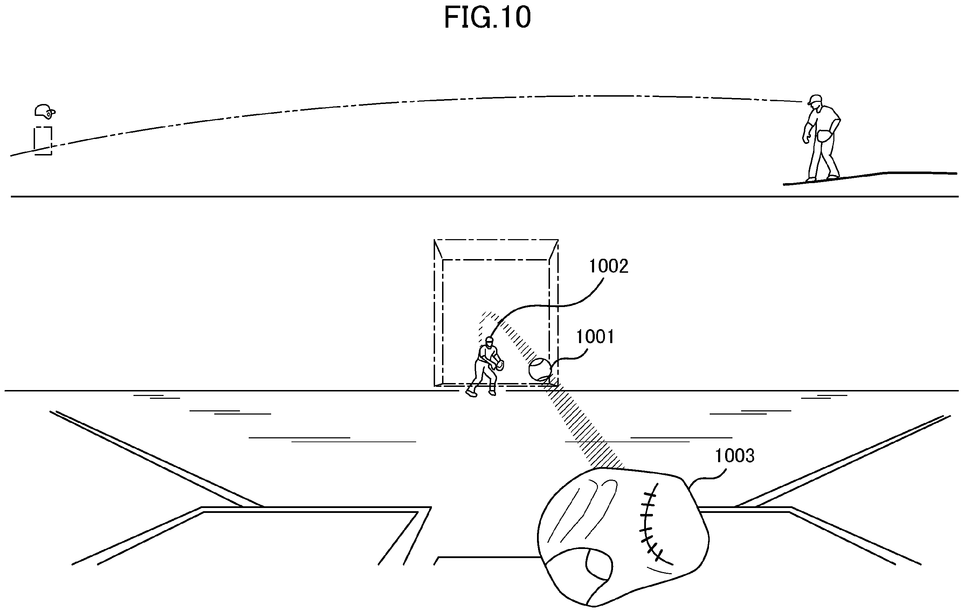

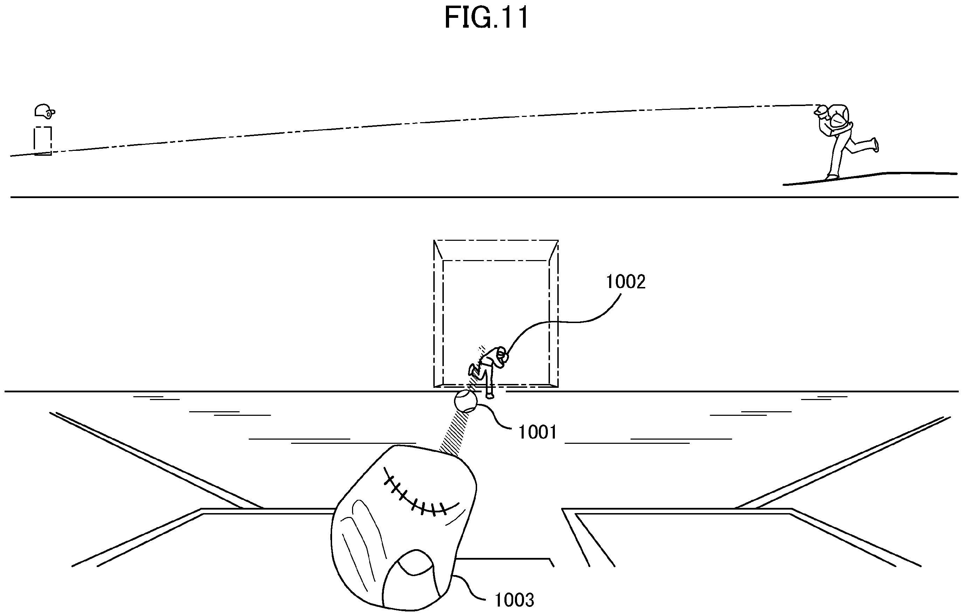

Figures 10 and 11 are each an example of a display screen in the measurement test for measurement of the three-dimensional cognitive ability based on the catching of a ball that is pitched, and an image, seen from the point of view of a catching person, of the pitching of a ball 1001 by a pitching person 1002 to a mitt 1003 that is a catching tool of the catching person and a background corresponding to a field is shown in a lower part of each of Figures 10 and 11. A moving image including the image shown in the lower part of Figure 10 or 11 is displayed on the electronic displays 104. A state of the ball that is pitched, as seen from side, is shown in upper parts of Figures 10 and 11. Images in the upper parts of Figures 10 and 11 may be additionally displayed at upper parts of the images on the electronic displays 104 or do not have to be displayed. In Figure 10, the ball is pitched to the right in the batter's box at a slow speed and along a route that draws a concave down curve, and in Figure 11, the ball is pitched to the left in the batter's box at a fast speed and along a linear route.

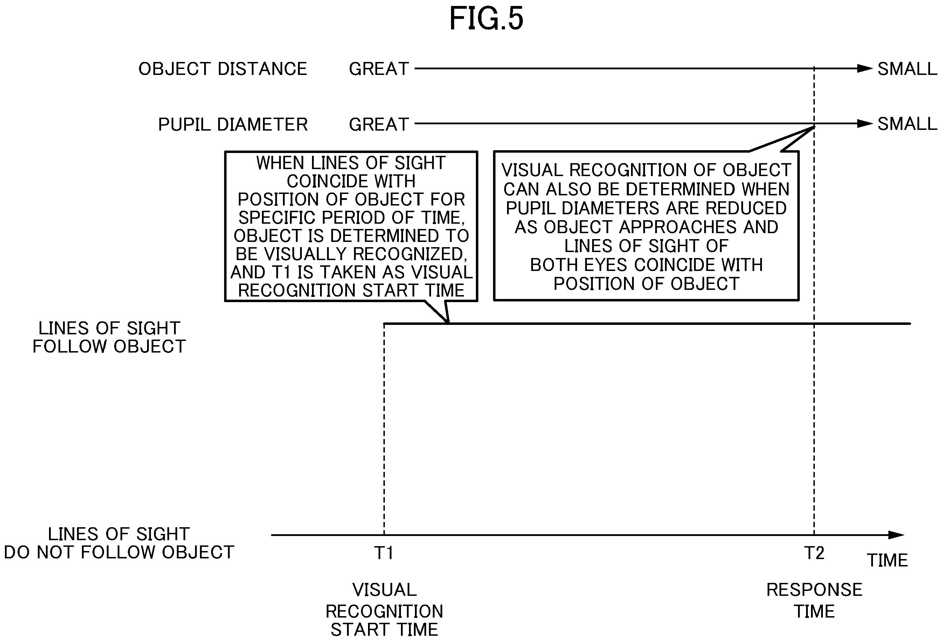

The visual recognition determination unit 101c computes a difference between the line-of-sight direction and the object position while the moving object is being displayed on the electronic display 104 (step S102). The visual recognition determination unit 101c acquires the position the object that is moving, in real time from the object position acquisition unit 101b. The visual recognition determination unit 101c acquires the line-of-sight directions of the left and right eyes from the line-of-sight/pupil sensors 105, and computes differences between the line-of-sight directions and the position of the object that is moving. Next, the visual recognition determination unit 101c determines whether the line-of-sight directions of both eyes coincide with the position of the object for a predetermined period of time or longer and the object is being followed, by determining whether the differences between the line-of-sight directions and the object position are equal to or smaller than a predetermined value for a predetermined period of time or longer (step S103). In this manner, the visual recognition determination unit 101c acquires the line-of-sight directions of the left and right eyes from the line-of-sight/pupil sensors 105, and determines whether the line-of-sight directions correctly face the position of the object that is moving and whether the line-of-sight directions of both eyes follow the position of the object. Whether the object that is moving is visually and spatially recognized by the measurement target person is thus determined. In the case where the line-of-sight directions of both eyes coincide with the position of the object for a certain period of time or longer, the visual recognition determination unit 101c determines that following is started, and records a time when the line-of-sight directions of both eyes start to coincide with the position of the object as a visual recognition start time T1. That is, the visual recognition start time T1 is a time after measurement is started based on pitching, from a time of start of movement of the object to when spatial recognition of the object by the measurement target person is determined. The visual recognition start time T1 indicates swiftness of visual recognition, and a smaller value can be evaluated to indicate swifter visual recognition. Figure 5 describes the visual recognition start time using a drawing. A graph in Figure 5 takes time as a horizontal axis, and a vertical axis indicates a state where the line of sight follows an object and a state where the line of sight does not follow the object. In this manner, the visual recognition determination unit 101c determines that an object is visually and spatially recognized by the measurement target person, in a case where the line-of-sight directions of both eyes coincide with and follow the position of the object for a predetermined period of time or longer.

Furthermore, the visual recognition determination unit 101c is also capable of determining that the object is visually and spatially recognized by the measurement target person, based not only on whether the line-of-sight directions follow the position of the object for a predetermined period of time or longer, but also by using reduction in the pupil diameters as an additional condition (this step is not shown in Figure 4). The distance to the object and the state of the pupil diameter are also shown in an upper part in Figure 5. In the case where the pupil diameters are reduced as the distance to the object is reduced due to the position of the object approaching in a point-of-view direction, the visual recognition determination unit 101c determines that the measurement target person is visually and spatially recognizing the object. It is noted that additional determination based on the pupil diameters is desirably performed when the object comes close to the point of view. Computation in step S102 of the difference between the line-of-sight direction and the object position and determination in step S 103 that the object is visually and spatially recognized may be taken as preconditions for determination, described later, of the three-dimensional cognitive ability based on the response of the measurement target person, and in this case, determination of the three-dimensional cognitive ability may be reliably performed based on visual and spatial recognition. However, it is also possible not to perform the steps before the determination of the three-dimensional cognitive ability based on the response of the measurement target person. In this case, the three-dimensional cognitive ability may be determined by a configuration and operation of a simpler system.

Next, the three-dimensional cognitive ability determination unit 101e calculates a distance between the object and the hand part based on the object position and the response of the measurement target person (step S104). The three-dimensional cognitive ability determination unit 101e acquires the position of the object that is moving, in real time from the object position acquisition unit 101b. The three-dimensional cognitive ability determination unit 101e identifies the position of the hand part at the tip of the arm based on the information about the position of the arm of the measurement target person acquired from the arm state sensor 106, and identifies a range of a position where the hand part can catch the object, by taking into account a size of the hand part. It is noted that in the case where the catching tool (such as a mitt or a glove) is worn on the hand part, the range of the position where the hand part can catch the object (a ball catching range) is identified by taking into account a size of the catching tool. Then, the three-dimensional cognitive ability determination unit 101e calculates the distance between the position of the object and the hand part (or the catching tool) until the object that is moving reaches the movement end position.

Next, the three-dimensional cognitive ability determination unit 101e determines whether a smallest distance between the object and the hand part that is calculated falls to or below a predetermined value before the object that is moving reaches the movement end position (step S105). When the distance between the object and the hand part (or the catching tool) falls to or below the predetermined value, it is determined that the object is caught by the hand part (or the catching tool) and that the ball is successfully caught, and the operation flow proceeds to step S106. That is, it is determined that the response of the measurement target person correctly matches the position of the object. The three-dimensional cognitive ability determination unit 101e records the smallest distance between the hand part and the object as a smallest distance L1, and records a time when it is determined that the ball is successfully caught as a response time T2. The smallest distance L1 is an indicator of accuracy of response, and the response is evaluated to be more accurate, the smaller the value. The response time T2 is an indicator of swiftness of response, and the response is evaluated to be swifter, the smaller the value. The response time T2 is described in Figure 5. The response time T2 is a time between start of movement of the object and when the distance between the hand part and the object reaches the smallest distance. In this manner, a determination result that there is no problem with the three-dimensional cognitive ability of the measurement target person may be obtained based on the successful catching of the ball. It is noted that determination may also be performed from a plurality of standpoints for more precise determination. For example, parameters such as the visual recognition start time T1, the smallest distance L1 between the hand part and the object, and the response time T2 (hereinafter referred to as "response parameters") may be acquired, and the three-dimensional cognitive ability of the measurement target person may be quantitatively calculated based thereon. Specifically, a score may be calculated based on a numerical value of a response parameter by associating the numerical value of each response parameter and the score with each other, and a weight is set in relation to each response parameter, and the three-dimensional cognitive ability may be quantified by totaling results obtained by multiplying the scores by respective weights, for example, and the three-dimensional cognitive ability determination unit 101e may thus calculate and output the quantified three-dimensional cognitive ability. A value of the weight may take a greater value, the greater influence the response parameter has on the determination result.

In the case where the distance between the object and the hand part (or the catching tool) does not fall to or below the predetermined value even when the object that is moving reaches the movement end position (in the case where the object does not enter the ball catching range), the three-dimensional cognitive ability determination unit 101e determines the unsuccessful catching of the ball, and the operation flow proceeds to step S107.

In the case where it is determined that the ball is successfully caught, the three-dimensional cognitive ability determination unit 10 1e counts up the number of successes by adding one to number of successes N (step S106). Then, the operation flow proceeds to step S107. Next, the three-dimensional cognitive ability determination unit 101e determines whether the measurement test is performed a predetermined number of times of measurement (step S107). When the measurement test is not performed a predetermined number of times, the operation flow returns to step S101, and the measurement test is performed from the start. That is, movement of the object by the moving object display unit 101a, determination, by the visual recognition determination unit 101c, of whether the object is visually and spatially recognized by the measurement target person, and evaluation of the three-dimensional cognitive ability by the three-dimensional cognitive ability determination unit 101e are repeated a predetermined number of times of measurement. The predetermined number of times of measurement is a number that is large enough that the number of successes is meaningful in evaluation but that is not excessively burdensome, such as ten times. When the measurement test is performed the predetermined number of times of measurement in step S107, the operation flow proceeds to step S108.

The three-dimensional cognitive ability determination unit 101e determines the three-dimensional cognitive ability based on results of the measurement tests, and outputs a determination result (step S108). A measurement value may be output as it is as the determination result. For example, the three-dimensional cognitive ability determination unit 101e may output the number of times the response is determined to correctly match the position of the object. Furthermore, instead of the number of times, a rate of success that is obtained by dividing the number of successes by the number of times of measurement may be output as the determination result. Moreover, a value (an average value) of each response parameter may be output as the determination result, in combination with the number of successes.

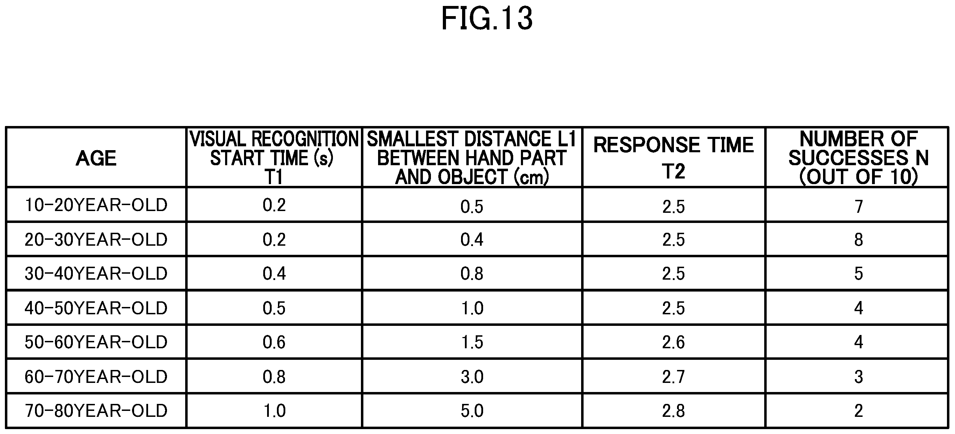

The three-dimensional cognitive ability determination unit 101e may also output, as the determination result, a result of comparing a measurement value with an expected value that is age-based. An expected value is an average of measurement values obtained by performing measurement for a large number of persons, and is a value that is expected from a standard measurement target person. An expected value that is age-based is an expected value where persons in a predetermined age group are taken as a parent population. Figure 13 shows an example of a table of expected values of response parameters and the number of successes based on age. Specifically, Figure 13 shows expected values, based on age, of the visual recognition start time T1, the smallest distance L1 between the hand part and the object, the response time T2, and the number of successes N. Data indicated in the table is stored as the background information data 103b, and is referred to by the three-dimensional cognitive ability determination unit 101e. It is noted that data of standard deviation may also be stored in addition to the expected values. The three-dimensional cognitive ability determination unit 101e receives input of age of the measurement target person, and acquires the expected values corresponding to the age from the background information data 103b, and may output, as the determination result, comparison results between the expected values and the measurement values. As the comparison result, the measurement value and the expected value may be output together in relation to each response parameter, or a ratio between the two may be output, or a deviation value may be calculated and output by using the data of standard deviation.

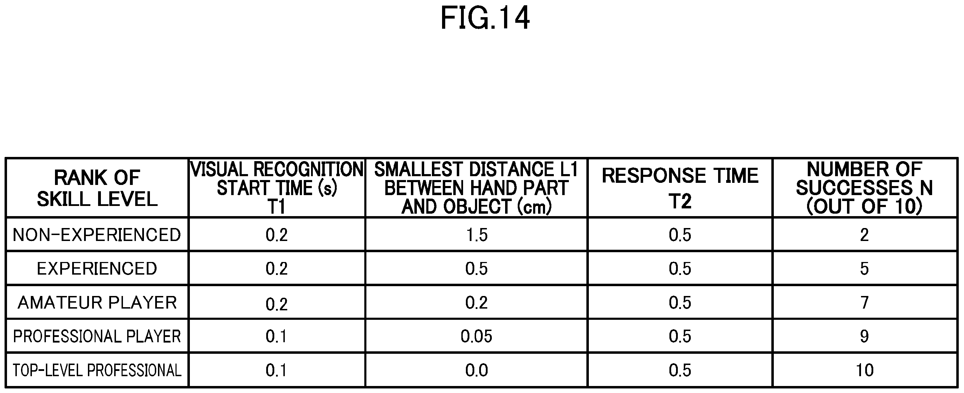

The three-dimensional cognitive ability determination unit 101e may also output, as the determination result, a result of comparing a measurement value with an expected value that is based on rank of skill level. Figure 14 shows an example of a table of expected values of response parameters and the number of successes based on the rank of skill level. Specifically, Figure 14 shows expected values, based on the rank of skill level, of the visual recognition start time T1, the smallest distance L1 between the hand part and the object, the response time T2, and the number of successes N. An expected value that is based on the rank of skill level is an expected value where persons in each rank of skill level are taken as a parent population. The rank of skill level here is, in the case of catching a ball, a rank of skill level in baseball, and the skill level may be classified into a person with no experience, a person with experience, an amateur player, a professional player, and a top-level professional, for example. Data indicated in the table is stored as the background information data 103b, and is referred to by the three-dimensional cognitive ability determination unit 101e. The three-dimensional cognitive ability determination unit 101e may quantify degrees of skill by comparing the measurement values of the measurement target person with the expected values that are based on the ranks of skill level and by identifying the rank of skill level the measurement values of the measurement target person are closest to, and may output the same as the determination result.

As described above, the three-dimensional cognitive ability determination unit 10 1e may quantify the three-dimensional cognitive ability from various standpoints, and may output the same as the determination result. That is, as the determination result, the three-dimensional cognitive ability determination unit 101e may output measurement values such as the number of successes, the rate of success, and the response parameters (the visual recognition start time T1, the smallest distance L1 between the hand part and the object, the response time T2). Furthermore, the three-dimensional cognitive ability determination unit 101e may output results of comparing the measurement values with the expected values that are age-based (in combination, ratio, deviation value, etc.). Furthermore, the three-dimensional cognitive ability determination unit 101e may output the rank of skill level closest to the measurement values.

### (First Modification: Hitting of Pitched Ball with Bat)

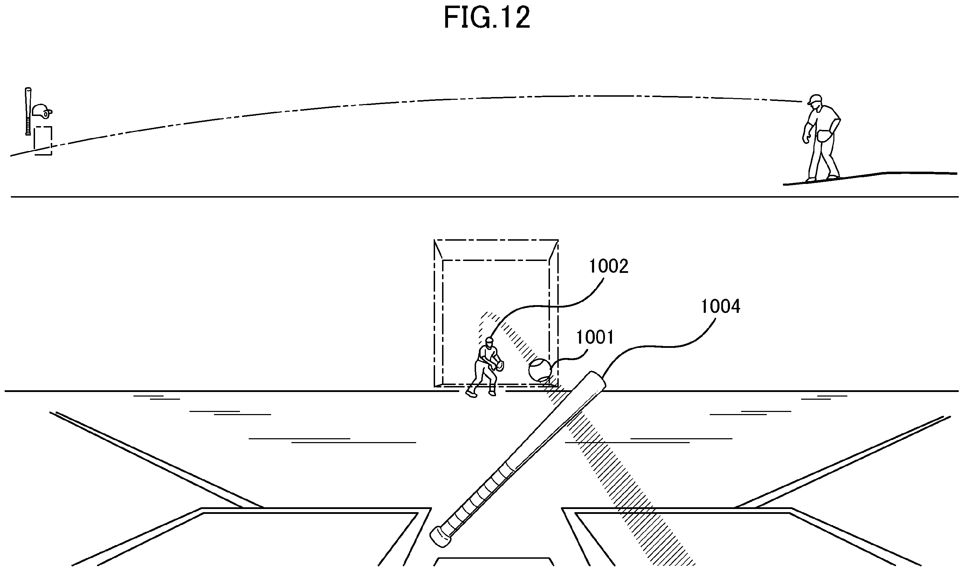

In the example described above, the three-dimensional cognitive ability is determined based on whether a pitched ball is caught or not, but the three-dimensional cognitive ability may also be determined in the same manner using various sports, driving operation and the like. Figure 12 shows an example of a display screen in a measurement test for measurement of the three-dimensional cognitive ability based on the hitting of a pitched ball with a bat. In the case of hitting a ball with a bat, as in the case of catching a pitched ball, an active response of the measurement target person is to bring the bat that is held on an arm that is a predetermined part of a body close to the ball as a moving object to hit the ball. The measurement target person brings the bat close to a position of the ball as a target. In the first modification, the three-dimensional cognitive ability is measured based on whether the ball is successfully hit with the bat, instead of being caught. A system for such measurement may have a configuration approximately the same as that of the three-dimensional cognitive ability evaluation system 100 described above, but the moving object display unit 101a uses the ball 1001 for baseball as the moving object, the pitching position of the pitching person (the pitcher) 1002 as the movement start position, the catching position of the catcher as the movement end position, the trajectory of the ball pitched by the pitcher as the predetermined movement route, and a point of view of a batter as the predetermined point of view. Then, the moving object display unit 101a identifies a position and a direction of a bat 1004 held on the arm, based on information about a position of the arm of the measurement target person acquired from the arm state sensor 106, and further displays the bat 1004 held on the arm. The three-dimensional cognitive ability determination unit 101e determines that the response of the measurement target person correctly matches the ball, in the case where a distance between a predetermined hitting region on the bat 1004 and the object falls to or below a predetermined distance. As the hitting region, a range within a contour of the bat 1004, or a range of a sweet spot on the bat 1004 may be used, for example. Moreover, a high score may be set for a position close to the sweet spot on the bat 1004.

### (Second Modification: Squash)

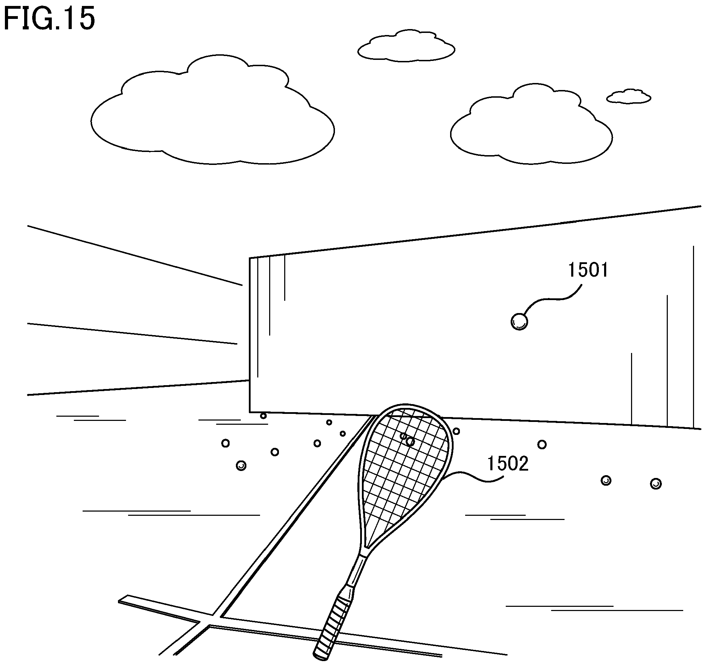

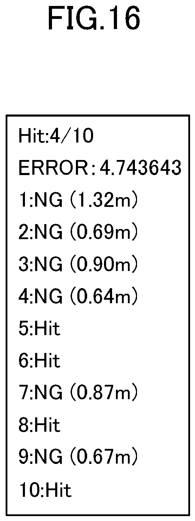

Furthermore, squash may also be used as a sport for determining the three-dimensional cognitive ability. In the case of squash, as in the case of catching a pitched ball, an active response of the measurement target person is to bring a racket held on an arm that is a predetermined part of a body close to a ball as a moving object. The measurement target person brings the racket close to a position of the ball as a target. Figure 15 shows an example of a display screen in a measurement test for measurement of the three-dimensional cognitive ability based on squash. In the second modification, the three-dimensional cognitive ability is measured based on whether the ball is successfully hit with a squash racket. A system for such measurement may have a configuration approximately the same as that of the three-dimensional cognitive ability evaluation system 100 described above, but the moving object display unit 101a uses a ball 1501 for squash as the moving object, a rebounding position on a wall as the movement start position, a position in front of a player as the movement end position, a trajectory of the ball 1501 rebounding off the wall as the predetermined movement route, a point of view of the player as the predetermined point of view, and a squash court as the background, and displays those mentioned above. Then, the moving object display unit 101a identifies a position and a direction of a racket 1502 held on the arm, based on information about a position of the arm of the measurement target person acquired from the arm state sensor 106, and further displays the racket 1502 held on the arm. The three-dimensional cognitive ability determination unit 101e determines that the response of the measurement target person correctly matches the ball, in the case where a distance between a predetermined hitting region on the racket 1502 and the object falls to or below a predetermined distance. As the hitting region, a range of a racket face of the racket 1502 may be used, for example. Moreover, a high score may be set for a position close to a center part of the racket face of the racket 1502. Figure 16 is a diagram showing an example of a measurement result of the measurement test based on squash. Here, 4/10 as the number of successes (Hit), an average distance (error) from a center of the racket at the time of success, and a smallest distance between the ball 1501 and the racket 1502 at the time of failure (NG) are indicated. The three-dimensional cognitive ability determination unit 101e may output those mentioned above as the measurement results.

### (Third Modification: Driving Simulation)

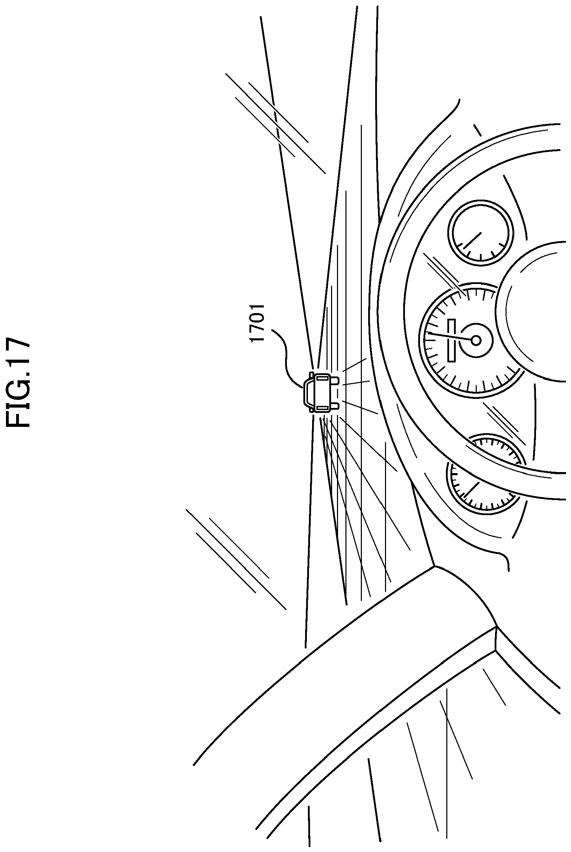

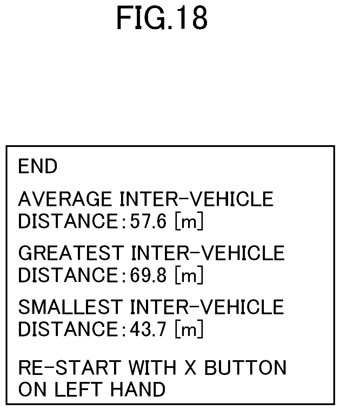

Furthermore, a driving operation of a vehicle may be used as a driving operation for determining the three-dimensional cognitive ability. In the case of the driving operation, to evaluate the three-dimensional cognitive ability based on a sense of distance, an operation of maintaining a constant distance to a preceding vehicle by an accelerator operation or the like may be used. In this case, an active response of the measurement target person is to maintain a constant distance between the preceding vehicle and a vehicle of the measurement target person. A difference between the preceding vehicle and the vehicle of the measurement target person is dynamically determined based on a position of the preceding vehicle, and a target of the response of the measurement target person is to maintain the difference at a constant value. Figure 17 shows an example of a display screen in a measurement test for measurement of the three-dimensional cognitive ability based on driving simulation. In the third modification, the three-dimensional cognitive ability is measured based on whether a distance to a preceding vehicle 1701 is maintained constant in the driving simulation. A system for such measurement may have a configuration approximately the same as that of the three-dimensional cognitive ability evaluation system 100 described above, but the moving object display unit 101a displays the preceding vehicle 1701 as the moving object whose speed changes within a predetermined range, at a position (a departure position) at a size of distance/depth corresponding to a time of start of measurement, with a road and a dashboard of the vehicle of the measurement target person as a background, calculates a speed and a position of the vehicle of the measurement target person according to an accelerator position, and changes the distance to the preceding vehicle 1701 based on a difference to the position of the preceding vehicle 1701. As the accelerator position, a level of pressure according to a position of an accelerator pedal that is stepped on by a foot of the measurement target person may be input based on information about a position of the foot acquired from the arm state sensor 106 attached to the foot. Alternatively, the accelerator position may be input by preparing a control device including an accelerator pedal and connected to the three-dimensional cognitive ability evaluation system 100 and acquiring therefrom information about the level of pressure on the accelerator pedal. Moreover, the accelerator position may be input using a dial or a lever that is operated with hand. Moreover, in addition to the accelerator pedal, stepping on a brake pedal may also be distinguished, and the speed may be reduced according to a level of pressure on the brake pedal. In the case where the distance to the preceding vehicle 1701 is within a predetermined range or close to a constant value, the three-dimensional cognitive ability determination unit 101e may determine the three-dimensional cognitive ability of the measurement target person to be normal. Furthermore, the smaller a difference between a greatest distance and a smallest distance between the position of the preceding vehicle and the position of the vehicle of the measurement target person identified by the accelerator operation, the more accurately the constant distance is determined to be maintained, and thus, a higher three-dimensional cognitive ability may be determined for the measurement target person. Figure 18 is a diagram showing an example of a measurement result of the measurement test of the three-dimensional cognitive ability based on driving simulation. Here, an average inter-vehicle distance, a greatest inter-vehicle distance, and a smallest inter-vehicle distance are shown. The three-dimensional cognitive ability determination unit 101e may output these as the determination result.

### (Fourth Modification: Drone Landing Operation)

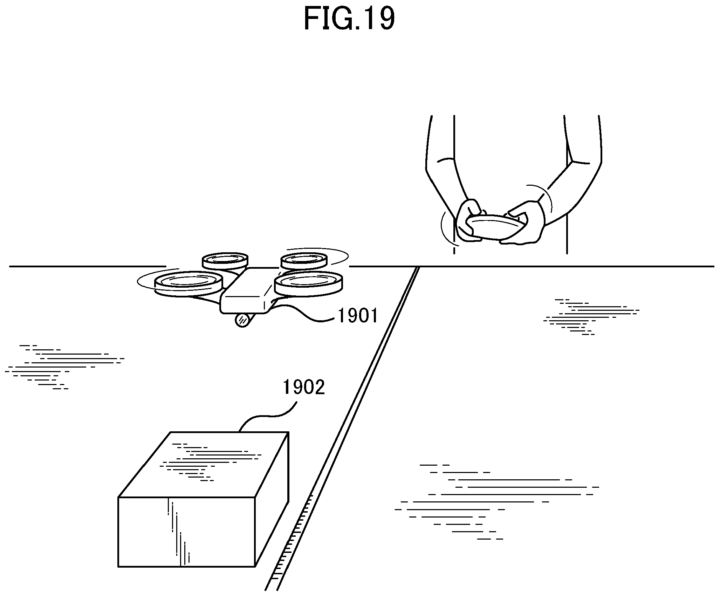

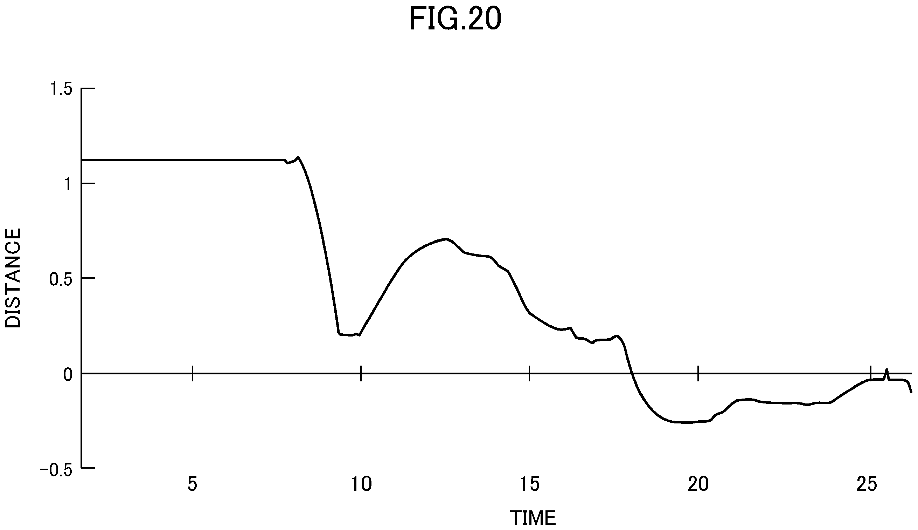

Furthermore, an operation of an operation target object such as a drone, such as a landing operation of the drone, may be used as a driving operation for determining the three-dimensional cognitive ability. In this case, an active response of the measurement target person is to bring an operation target object that is a drone as the moving object, to a landing platform that is a predetermined location. A target of the response of the measurement target person is to reduce a difference between a position of the drone and a position of the landing platform that is dynamically determined according to the position of the drone. In the fourth modification, the three-dimensional cognitive ability is measured by degrees of skill in the landing operation through the actual maneuvering of the drone. A system for such measurement may be realized by an information terminal such as a smartphone that is connected to a predetermined sensor, without using the virtual reality headset including the electronic displays, as in the case of the three-dimensional cognitive ability evaluation system 100 described above. With the information terminal, functional blocks corresponding to the object position acquisition unit 101b, the response input unit 101d, and the three-dimensional cognitive ability determination unit 101e of the three-dimensional cognitive ability evaluation system 100 are implemented through execution of predetermined programs by a processor of the information terminal. In this case, the moving object is the drone that is an operation target object to be moved being operated by the measurement target person, from a departure position to a target position, and the response input unit 101d receives the position of the drone as an input of the response, and the three-dimensional cognitive ability determination unit 101e evaluates the three-dimensional cognitive ability of the measurement target person based on a difference between the position of the drone and the target position. Figure 19 shows an image view of a measurement test for measurement of the three-dimensional cognitive ability based on the drone landing operation. Specifically, the three-dimensional cognitive ability is measured based on whether the measurement target person is able to land a drone 1901 on a landing platform 1902 by actually visually recognizing and maneuvering the drone 1901, and on degrees of skill at the time. A center part of the landing platform 1902 is the target position, and a high degree of skill in the landing operation is determined when the drone 1901 is landed at a position close to the target position. After the drone 1901 starts moving from the departure position, the object position acquisition unit 101b acquires a real-time position of the drone 1901. The position of the drone 1901 is at least a one-dimensional position on a straight line connecting the position of the measurement target person and the position of the landing platform 1902. A two-dimensional position where a position in an orthogonal direction of the straight line on a horizontal plane is added, or a three-dimensional position where a position in a height direction is further added may also be used as the position of the drone 1901. The position of the drone 1901 may be identified by capturing the drone 1901 by a camera or the like and by identifying the position from an image, or the position of the drone 1901 may be identified by a distance sensor. Furthermore, the position of the drone 1901 may be acquired by attaching a position sensor to the drone 1901. The position of the drone 1901 is input to the response input unit 101d as the response of the measurement target person. The three-dimensional cognitive ability determination unit 101e has a position of the center part (the target position) of the landing platform 1902 stored therein, and determines in real-time a difference (a distance) from the position of the drone 1901 input to the response input unit 101d. The three-dimensional cognitive ability determination unit 101e identifies success/failure of landing, a size of difference between the drone 1901 and the landing platform 1902, and the like based on the real-time difference (distance) between the position of the drone 1901 and the position of the landing platform 1902, and thereby performs determination of the three-dimensional cognitive ability. That is, when the distance between the drone 1901 and the landing platform 1902 is within a range of a size of the landing platform 1902, and movement of the drone 1901 is stopped within the range, the landing of the drone 1901 on the landing platform 1902 can be determined, and when the drone 1901 is landed at a position close to the center part of the landing platform 1902, degrees of skill in the landing operation can be determined. Figure 20 is a diagram showing an example of a measurement result of the measurement test based on the drone landing operation. Here, a graph showing a manner of change in the distance between the drone 1901 and the landing platform 1902 over time is shown. The three-dimensional cognitive ability determination unit 101e may output success/failure of landing as the determination result, by determining whether landing is successful with the distance from the landing platform being within a predetermined range. Moreover, the three-dimensional cognitive ability determination unit 101e may output the distance between the drone 1901 and the center part (the target position) of the landing platform 1902 in the case of successful landing, as the determination result indicating a degree of skill in the landing operation. The three-dimensional cognitive ability determination unit 101e may also output, as the determination results, a smallest distance and an average distance between the drone 1901 and the center part of the landing platform 1902 within a range of a predetermined period of time.

### Industrial Applicability

The present invention quantifies the three-dimensional cognitive ability, and may thus be used in fields of medical treatment, preventive medicine, medical equipment and the like where the three-dimensional cognitive ability and cognitive function of a target person needs to be objectively grasped.

### Reference Signs List

100: three-dimensional cognitive ability evaluation system
101: processor
101a: moving object display unit
101b: object position acquisition unit
101c: visual recognition determination unit
101d: response input unit
101e: three-dimensional cognitive ability determination unit
102: RAM
103: memory
103a: three-dimensional cognitive ability evaluation program
103b: background information data
104: electronic display
105: line-of-sight/pupil sensor
106: arm state sensor
107: interface
1001: ball
1002: pitching person
1003: mitt
1004: bat
1501: ball
1502: racket
1701: preceding vehicle
1901: drone
1902: landing platform
L1: smallest distance
N: number of successes
T1: visual recognition start time
T2: response time

## Claims

1. A three-dimensional cognitive ability evaluation system for evaluating a three-dimensional cognitive ability based on a response of a measurement target person to a moving object, the three-dimensional cognitive ability evaluation system comprising:
an object position acquisition unit configured to acquire information about a position of the moving object, the information enabling identification of a distance between the moving object and the measurement target person;
a response input unit configured to receive an input of an active response of the measurement target person taken in response to a position of the object that is recognized by the measurement target person; and
a three-dimensional cognitive ability determination unit configured to evaluate the three-dimensional cognitive ability of the measurement target person by determining whether the position of the object that is acquired and the response that is input correctly match.

2. The three-dimensional cognitive ability evaluation system according to claim 1, wherein the three-dimensional cognitive ability determination unit evaluates the three-dimensional cognitive ability of the measurement target person based on a positional correspondence relationship between positions of the object that are acquired and positions that are identified based on the response, within a predetermined range of time.

3. The three-dimensional cognitive ability evaluation system according to claim 2, wherein the positional correspondence relationship includes at least one of
a smallest distance between the positions of the object and the positions that are identified based on the response,
an average distance between the positions of the object and the positions that are identified based on the response, and
a difference between a greatest distance and the smallest distance between the positions of the object and the positions that are identified based on the response.

4. The three-dimensional cognitive ability evaluation system according to claim 1, wherein
the moving object is an operation target object that is moved from a departure position toward a target position by an operation by the measurement target person,
the response input unit receives a position of the operation target object as the input of the response, and
the three-dimensional cognitive ability determination unit evaluates the three-dimensional cognitive ability of the measurement target person based on a difference between the position of the operation target object and the target position.

5. The three-dimensional cognitive ability evaluation system according to any one of claims 1 to 3, further comprising:
an eyeball state sensing unit configured to sense line-of-sight directions of both eyes of the measurement target person; and
a visual recognition determination unit configured to determine whether the object is visually and spatially recognized by the measurement target person, by determining whether the line-of-sight directions correctly match the position of the object that is moving, wherein
the three-dimensional cognitive ability determination unit evaluates the three-dimensional cognitive ability of the measurement target person by determining, in a case where visual and spatial recognition of the object by the measurement target person is determined by the visual recognition determination unit, whether the response that is input correctly matches the position of the object that is acquired.

6. The three-dimensional cognitive ability evaluation system according to claim 5, wherein, in a case where the line-of-sight directions of both eyes are each determined to coincide with the position of the moving object, for a predetermined period of time or longer, the visual recognition determination unit determines that the object is visually recognized by the measurement target person.

7. The three-dimensional cognitive ability evaluation system according to claim 5 or 6, wherein
the eyeball state sensing unit further senses pupil diameters of both eyes of the measurement target person, and
in a case of further determining that the pupil diameters of the both eyes are gradually reduced as the position of the object moves closer to predetermined point of view, the visual recognition determination unit determines that the object is visually and spatially recognized by the measurement target person.

8. The three-dimensional cognitive ability evaluation system according to any one of claims 5 to 7, wherein
the moving object is provided in virtual reality,
the three-dimensional cognitive ability evaluation system further includes
a virtual reality headset including an electronic display for displaying a moving image in the virtual reality, and
a moving object display unit configured to cause the electronic display to display a moving image in which the object seen from a predetermined point of view in the virtual reality moves from a movement start position to a movement end position along a predetermined movement route in a direction of approaching the predetermined point of view, and
the object position acquisition unit acquires the position of the object in the virtual reality displayed by the movement object display unit.

9. The three-dimensional cognitive ability evaluation system according to claim 8, wherein
the response input unit continuously identifies a position of a predetermined part of a body of the measurement target person based on a signal from a sensor attached to the predetermined part of the body, where the position of the predetermined part of the body is input as the response,
the movement object display unit further causes an image of at least a part of the predetermined part of the body of the measurement target person to be displayed in the virtual reality on the electronic display, based on the position of the predetermined part of the body that is identified, and
the three-dimensional cognitive ability determination unit determines correct matching of the response in a case where a distance between a predetermined portion related to the predetermined part of the body and the object falls to or below a predetermined distance in a case where spatial recognition of the object by the measurement target person is determined by the visual recognition determination unit.

10. The three-dimensional cognitive ability evaluation system according to any one of claims 5 to 9, wherein the three-dimensional cognitive ability determination unit acquires three response parameters including a visual recognition start time from a movement start time of the object to when spatial recognition of the object by the measurement target person is determined, a smallest distance between a predetermined portion related to a predetermined part of a body and the object, and a response time from the movement start time of the object to when a distance between the predetermined portion related to the predetermined part of the body and the object reaches the smallest distance, and evaluates the three-dimensional cognitive ability of the measurement target person based on the response parameters.

11. The three-dimensional cognitive ability evaluation system according to claim 10, wherein the three-dimensional cognitive ability determination unit calculates scores based on numerical values of the response parameters, and evaluates the three-dimensional cognitive ability of the measurement target person based on a total of the scores that are multiplied by respective predetermined weights.

12. The three-dimensional cognitive ability evaluation system according to claim 10 or 11, wherein
movement of the object by the moving object display unit, determination by the visual recognition determination unit of whether the object is visually and spatially recognized by the measurement target person, and evaluation of the three-dimensional cognitive ability by the three-dimensional cognitive ability determination unit are repeated a predetermined number of times of measurement, and
the three-dimensional cognitive ability determination unit further outputs a number of times when the response is determined to correctly match the position of the object.

13. A three-dimensional cognitive ability evaluation apparatus for evaluating a three-dimensional cognitive ability based on a response of a measurement target person to a moving object, the three-dimensional cognitive ability evaluation apparatus comprising:
an object position acquisition unit configured to acquire information about a position of the moving object, the information enabling identification of a distance between the moving object and the measurement target person;
a response input unit configured to receive an input of an active response of the measurement target person taken in response to a position of the object that is recognized by the measurement target person; and
a three-dimensional cognitive ability determination unit configured to evaluate the three-dimensional cognitive ability of the measurement target person by determining whether the position of the object that is acquired and the response that is input correctly match,
the object position acquisition unit, the response input unit, and the three-dimensional cognitive ability determination unit being included in one housing.

14. A three-dimensional cognitive ability evaluation program being executed by a computer to cause the computer to implement a three-dimensional cognitive ability evaluation system for evaluating a three-dimensional cognitive ability based on a response of a measurement target person to a moving object, wherein the three-dimensional cognitive ability evaluation system includes
an object position acquisition unit configured to acquire information about a position of the moving object, the information enabling identification of a distance between the moving object and the measurement target person;
a response input unit configured to receive an input of an active response of the measurement target person taken in response to a position of the object that is recognized by the measurement target person; and
a three-dimensional cognitive ability determination unit configured to evaluate the three-dimensional cognitive ability of the measurement target person by determining whether the position of the object that is acquired and the response that is input correctly match.

15. A three-dimensional cognitive ability evaluation method for evaluating a three-dimensional cognitive ability based on a response of a measurement target person to a moving object, the three-dimensional cognitive ability evaluation method comprising:
an object position acquisition step of acquiring information about a position of the moving object, the information enabling identification of a distance between the moving object and the measurement target person;
a response input step of receiving an input of an active response of the measurement target person taken in response to a position of the object that is recognized by the measurement target person; and
a three-dimensional cognitive ability determination step of evaluating the three-dimensional cognitive ability of the measurement target person by determining whether the position of the object that is acquired and the response that is input correctly match.
